(19) Europäisches Patentamt
European Patent Office
Office européen des brevets

(11) Veröffentlichungsnummer: **0 169 993 B1**

(12) **EUROPÄISCHE PATENTSCHRIFT**

(45) Veröffentlichungstag der Patentschrift: **28.12.88**

(51) Int. Cl.⁴: **C 07 D 401/04**, C 07 D 409/14, A 61 K 31/495, C 07 D 407/14, C 07 D 215/56

(21) Anmeldenummer: **85106252.1**

(22) Anmeldetag: **22.05.85**

(54) **7-(3-Aryl-1-piperazinyl)-sowie 7-(3-Cyclohexyl-1-piperazinyl)-3-chinoloncarbonsäuren.**

(30) Priorität: **04.06.84 DE 3420798**

(43) Veröffentlichungstag der Anmeldung: **05.02.86 Patentblatt 86/6**

(45) Bekanntmachung des Hinweises auf die Patenterteilung: **28.12.88 Patentblatt 88/52**

(84) Benannte Vertragsstaaten: **AT BE CH DE FR GB IT LI LU NL SE**

(56) Entgegenhaltungen:
**EP-A- 0 049 355**
**EP-A- 0 113 091**
**EP-A- 0 113 093**
**EP-A- 0 126 355**
**US-A- 4 398 029**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Zeiler, Hans-Joachim, Dr., Elsbeeker Strasse 46, D-5620 Velbert 15 (DE)**
Erfinder: **Metzger, Karl-Georg, Dr., Pahlkestrasse 75, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue 1-Cyclopropyl-1,4-dihydro-4-oxo-3-chinolincarbonsäuren, Verfahren zu ihrer Herstellung sowie diese enthaltende antibakterielle Mittel und Futterzusatzstoffe.

Es wurde gefunden, dass die neuen 1-Cyclopropyl-6-fluor-1,4-dihydro-4-oxo-7-(1-piperazinyl)-3-chinolincarbonsäuren der Formel (I)

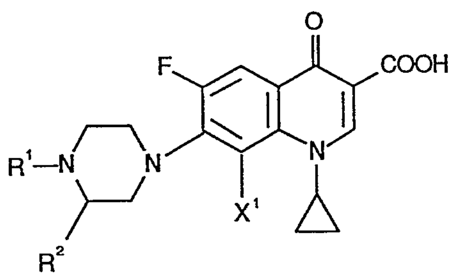

(I),

in welcher

$R^1$ Wasserstoff, gegebenenfalls durch Hydroxy, Methoxy, Amino, Dimethylamino, Halogen, wie beispielsweise Fluor oder Chlor, Cyano oder Alkoxycarbonyl mit ein oder zwei Kohlenstoffatomen Alkylteil substituiertes geradkettiges oder verzweigtes Alkyl mit 1 bis 4 Kohlenstoffatomen, Oxoalkyl mit bis zu 4 Kohlenstoffatomen, Phenacyl, Acyl mit 1 bis 4 Kohlenstoffatomen,

$R^2$ gegebenenfalls durch Halogen, wie beispielsweise Chlor, Brom oder Fluor, Methyl, Phenyl, Cyano, Hydroxy, Methoxy, Benzyloxy, Amino, Methylamino, Dimethylamino, Piperidino oder Nitro ein- bis dreifach substituiertes Phenyl und Cyclohexyl, Methylendioxy-phenyl, Methylendixycyclohexyl, Thienyl und

$X^1$ Wasserstoff oder Fluor

bedeuten, und deren pharmazeutisch verwendbaren Hydrate, Säureadditionssalze, Alkali- und Erdalkalisalze eine hohe antibakterielle Wirkung aufweisen.

Bevorzugt sind diejenigen Verbindungen der Formel (I), in denen

$R^1$ Wasserstoff, gegebenenfalls durch Hydroxy, Methoxy, Halogen, wie beispielsweise Fluor oder Chlor, Cyano oder Alkoxycarbonyl mit ein oder zwei Kohlenstoffatomen im Alkylteil substituiertes geradkettiges oder verzweigtes Alkyl mit 1 bis 4 Kohlenstoffatomen, Oxoalkyl mit bis zu 4 Kohlenstoffatomen, Phenacyl, Formyl oder Acetyl,

$R^2$ gegebenenfalls durch Chlor, Brom, Fluor, Methyl, Phenyl, Cyano, Hydroxy, Methoxy, Benzyloxy, Amino, Piperidino oder Nitro ein- bis dreifach substituiertes Phenyl und Cyclohexyl sowie Thienyl und

$X^1$ Wasserstoff oder Fluor bedeuten.

Besonders bevorzugt sind diejenigen Verbindungen der Formel (I), in denen

$R^1$ Wasserstoff, gegebenenfalls durch Hydroxy, Methoxy, Cyano oder Alkoxycarbonyl mit ein oder zwei Kohlenstoffatomen im Alkylteil substituiertes geradkettiges oder verzweigtes Alkyl mit 1 bis 3 Kohlenstoffatomen, Oxoalkyl mit bis zu 4 Kohlenstoffatomen, Phenacyl, Formyl oder Acetyl,

$R^2$ gegebenenfalls durch Chlor, Brom, Fluor, Methyl, Phenyl, Hydroxy, Methoxy, Benzyloxy, Amino, Piperidino oder Nitro ein- bis dreifach substituiertes Phenyl und Cyclohexyl sowie Thienyl und

$X^1$ Wasserstoff oder Fluor bedeuten.

Weiterhin wurde gefunden, dass man die Verbindungen der Formel (I) erhält, wenn man 1-Cyclopropyl-7-fluor-1,4-dihydro-4-oxo-3-chinolincarbonsäuren der Formel (II)

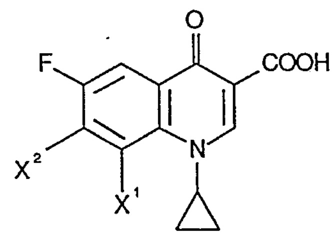

(II),

in welcher

$X^1$ die oben angegebene Bedeutung hat und

$X^2$ für Halogen, insbesondere Fluor oder Chlor steht, mit Piperazinen der Formel (III)

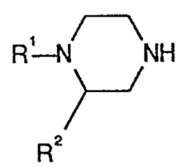

(III)

in welcher

$R^1$ und $R^2$ die oben angegebene Bedeutung haben, gegebenenfalls in Gegenwart von Säurebindern umsetzt (Methode A).

Die erfindungsgemässen Verbindungen der Formel (I) können auch erhalten werden, indem man eine 7-(1-Piperazinyl)-3-chinoloncarbonsäure der Formel (IV)

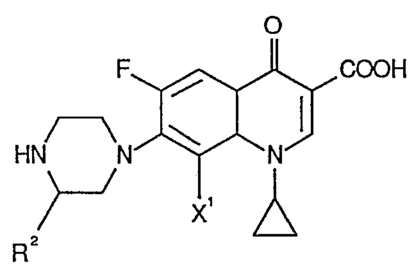

(IV)

in welcher $R^2$ und $X^1$ die oben angegebene Bedeutung haben, mit Verbindungen der Formel (V)

$R^1$—X (V)

in welcher

$R^1$ die oben angegebene Bedeutung hat, jedoch nicht Wasserstoff sein kann, und

X Fluor, Chlor, Brom, Jod, Hydroxy, Acyloxy, Phenoxy oder 4-Nitrophenoxy bedeutet, gegebenenfalls in Gegenwart von Säurebindern umsetzt (Methode B).

Man erhält die erfindungsgemässen Verbindungen der Formel (I) auch, wenn man eine 7-(1-Piperazinyl)-3-chinoloncarbonsäure der Formel

(IV) mit einem Michael-Acceptor der Formel (VI)

$R^3$—CH=$CH_2$ (VI)

in der $R^3$ für CN, $CH_3CO$ oder $COOR^4$ steht, wobei $R^4$ Methyl oder Ethyl bedeutet, umsetzt (Methode C).

Verwendet man bei der Umsetzung nach Methode A 2-Phenyl-piperazin und 1-Cyclopropyl-6,7,8-trifluor-1,4-dihydro-4-oxo-3-chinolincarbonsäure als Ausgangsstoffe, so kann der Reaktionsablauf durch das folgende Formelschema wiedergegeben werden:

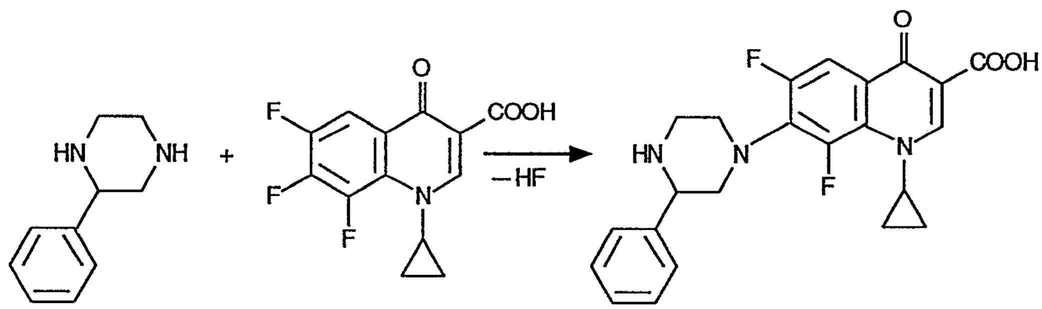


Verwendet man bei der Umsetzung nach Methode B Ethyljodid und 1-Cyclopropyl-6,8-difluor-1,4-dihydro-4-oxo-7-(3-phenyl-1-piperazinyl)-3-chinolincarbonsäure als Ausgangsstoffe, so kann der Reaktionsablauf durch das folgende Formelschema wiedergegeben werden.

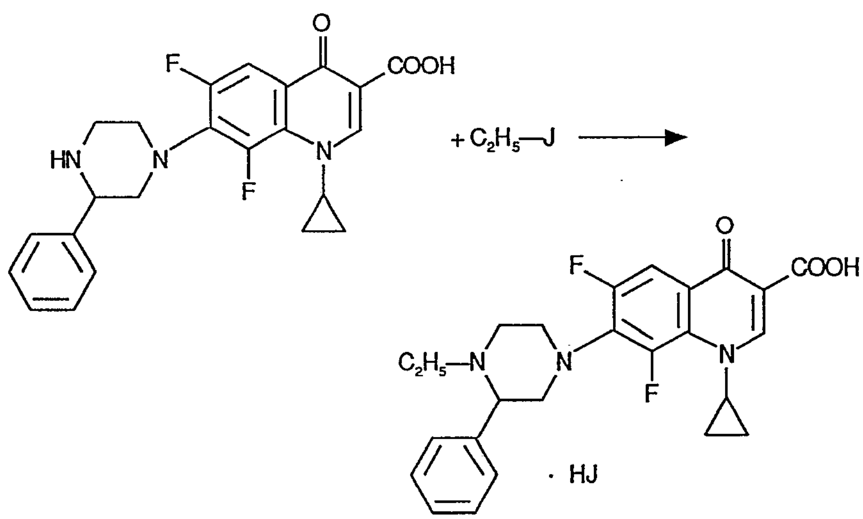


Verwendet man beispielsweise bei der Umsetzung nach Methode C 1-Cyclopropyl-6-fluor-1,4-dihydro-4-oxo-7-(3-phenyl-1-piperazinyl)-3-chinolincarbonsäure und Methyl-vinyl-keton als Ausgangsverbindungen, so kann der Reaktionsablauf durch das folgende Formelschema wiedergegeben werden:

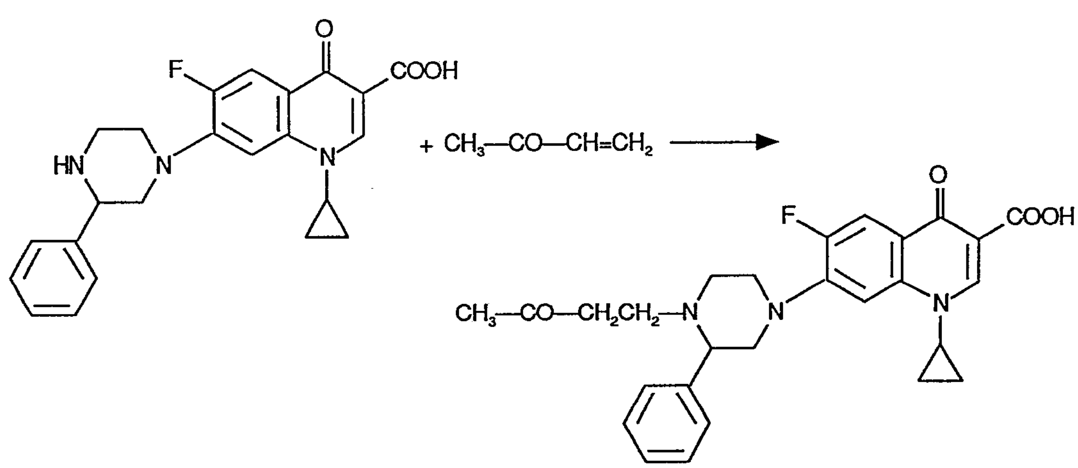

Die als Ausgangsverbindungen verwendeten Chinoloncarbonsäuren (II) sind teilweise bekannt (vergleiche DE-OS 3 142 854: 7-Chlor-1-cyclopropyl-6-fluor-1,4-dihydro-4-oxo-3-chinolincarbonsäure) oder lassen sich auf folgendem Weg herstellen:

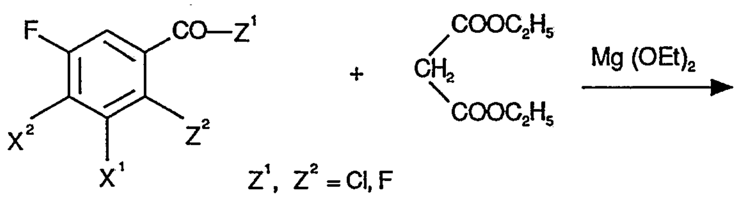


(1) (2)

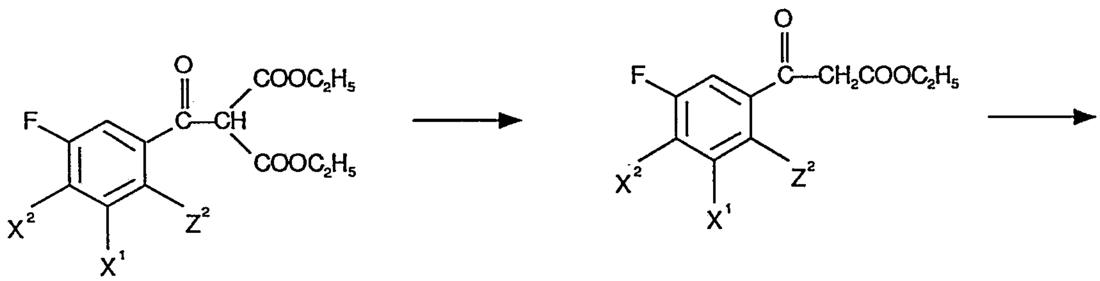

(3) (4)

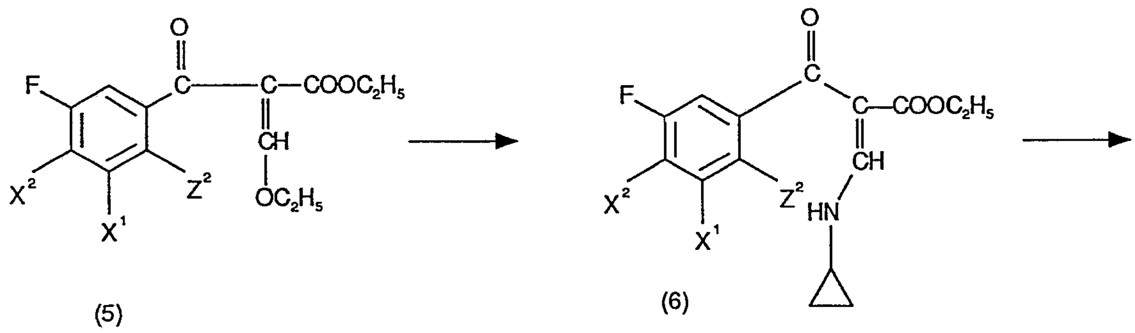

(5) (6)

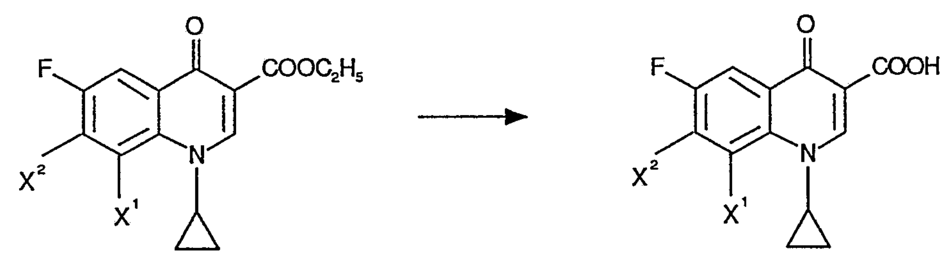

(7) (II)

Danach wird Malonsäurediethylester (2) in Gegenwart von Magnesiumethylat mit den entsprechenden Benzoylfluorid bzw. -chlorid (1) zum Aroylmalonester (3) acyliert (Organicum, 3. Aufl. 1964, S. 438).

Durch partielle Verseifung und Decarboxylierung von (3) in wässrigem Medium mit katalytischen Mengen Schwefelsäure oder p-Toluolsulfonsäure erhält man in guter Ausbeute den Aroylessigsäureethylester (4), der mit Orthoameisensäure-triethylester/Acetanhydrid in den entsprechenden 2-Benzoyl-3-ethoxy-acrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300°C, bevorzugt 80 bis 180°C durchgeführt.

Als Verdünnungsmittel können Dioxan, Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstufe Kaliumtert.-Butanolat, Butyl-lithium, Lithium-phenyl, Phenylmagnesiumbromid, Natriummethylat, Natriumhydrid, Natrium- oder Kalium-carbonat in Betracht. Wenn Fluorwasserstoff abgespalten werden soll ($Z^2$ = F), haben sich auch Kalium- oder Natrium-fluorid als besonders geeignet erwiesen. Es kann vorteilhaft sein, einen Überschuss von 10 Mol-% an Base einzusetzen.

Die in dem letzten Schritt erfolgende Esterhydrolyse von (7) unter basischen oder sauren Bedingungen führt zur entsprechenden Cyclopropyl-6-fluor-1,4-dihydro-4-oxo-3-chinolincarbonsäure (II).

Das als Ausgangsmaterial für diesen Syntheseweg verwendete 2,3,4,5-Tetrafluorbenzoylchlorid (1) ($X^1$ = $X^2$ = $Z^2$ = F, $Z^1$ = Cl) wurde aus der literaturbekannten 2,3,4,5-Tetrafluor-benzosäure [G.G. Yakobson, V.N. Odinokov und N.N. Vorozhtsov Jr., Zh. Obsh. Khim. 36, 139 (1966)] mit Thionylchlorid auf übliche Weise erhalten. Es besitzt einen Siedepunkt von 75–80°C/17 mbar. Das 2,3,4,5-Tetrafluorbenzoylfluorid besitzt einen Siedepunkt von 46 bis 47°C/20 mbar ($n_D^{20}$: 1,4375).

Analog wurde das als Ausgangsprodukt verwendete 2,4,5-Trifluorbenzoylfluorid (1) ($X^1$ = H, $X^2$ = $Z^1$ = $Z^2$ = F) aus der literaturbekannten 2,4,5-Trifluorbenzoesäure [I.J. DeGraw, M. Corey u. W.A. Skinner, J. Chem. Eng. Data 13, 587 (1968)] hergestellt. Es besitzt einen Siedepunkt von 53–56°/18 mbar ($n_D^{20}$: 1,4546).

Die als Ausgangsstoffe verwendeten 2-substituierten Piperazine (III) sind bekannt oder können nach literaturbekannten Verfahren erhalten werden [zum Beispiel: US 4 166 180; J. Med. Chem. 26, 1116 (1983)]. Als Beispiele seien genannt:

2-Phenylpiperazin, 2-(4-Chlorphenyl)-piperazin, 2-(4-Fluorphenyl)-piperazin, 2-(4-Bromphenyl)-piperazin, 2-(4-Methylphenyl)-piperazin, 2-(4-Biphenylyl)-piperazin, 2-(4-Methoxyphenyl)-piperazin, 2-(4-Benzyloxyphenyl)-piperazin, 2-(4-Hydroxyphenyl)-piperazin, 2-(3-Hydroxyphenyl)-piperazin, 2-(2-Hydroxyphenyl)-piperazin, 2-(4-Nitrophenyl)-piperazin, 2-(3-Nitrophenyl)-piperazin, 2-(4-Aminophenyl)-piperazin, 2-(4-Piperidinophenyl)-piperazin, 2-(3,4-Dimethoxyphenyl)-piperazin, 2-(3,4,5-Trimethoxyphenyl)-piperazin, 2-(3,4-Dimethoxy-6-methyl)-piperazin, 2-(3,4-Methylendioxyphenyl)-piperazin, 2-(4-Cyanophenyl)-piperazin, 2-(2-Thienyl)-piperazin.
Durch katalytische Hydrierung der 2-Aryl-piperazine werden gemäss einem eigenen Vorschlag vom gleichen Tage die entsprechenden 2-Cyclohexylpiperazine erhalten; zum Beispiel 2-Cyclohexylpiperazin (Schmelzpunkt 82–83°C).

Die als Ausgangsstoffe verwendeten Verbindungen der Formel (V) sind bekannt. Als Beispiele seien genannt:
Methyliodid, Methylbromid, Ethyliodid, Ethylbromid, Ethylchlorid, 2-Hydroxyethylchlorid, 3-Hydroxypropylchlorid, 4-Hydroxybutylchlorid, n-Propylbromid, i-Propyliodid, n-Butylbromid, i-Butylbromid, sek.-Butylchlorid, Ameisensäure, Ameisensäureessigsäureanhydrid, Essigsäureanhydrid oder Acetylchlorid.

Die erfindungsgemäss verwendbaren Verbindungen der Formel (VI) sind bekannt. Als Beispiele seien genannt: Acrylnitril, Methylvinylketon, Acrylsäuremethylester, Acrylsäureethylester.

Die Umsetzung von (II) mit (III) gemäss Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]octan (DABCO), überschüssiges Amin (III) oder 1,8-Diazabicyclo[5,4,0undec-7-en (DBU).

Die Reaktionstemperaturen können in einem grösseren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens setzt man auf 1 Mol der Carbonsäure (II) 1 bis 5 Mol, vorzugsweise 1 bis 3 Mol des Piperazins (III) ein.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2,2,2]octan (DABCO) oder 1,8-Diazabicyclo-[5,4,0]undec-7-en (DBU).

Die Reaktionstemperaturen können in einem grösseren Bereich variiert werden. Im allgemeinen arbeitet man zwischen etwa 20 und etwa 180°C, vorzugsweise zwischen 40 und 110°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens gemäss Methode B setzt man auf 1 Mol der Verbindung (IV) 1 bis 4 Mol, vorzugsweise 1 bis 1,5 Mol der Verbindung (V) ein.

Die Umsetzung von (I) mit (VI) (Methode C) wird in einem Verdünnungsmittel wie N,N-Dimethylformamid, Dioxan, Tetrahydrofuran, Pyridin, Wasser oder auch in Mischungen dieser Verdünnungsmittel durchgeführt. Die Reaktionstemperaturen können in einem grösseren Bereich variiert werden. Im allgemeinen arbeitet man zwischen etwa 0°C und etwa 140°C, vorzugsweise zwischen 10° und 100°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemässen Verfahrens setzt man auf 1 Mol der Verbindung (I) 1 bis 5 Mol, vorzugsweise 1 bis 2 Mol, der Verbindung (VI) ein.

Als neue Wirkstoffe seien ausser den in den Beispielen aufgeführten Verbindungen im einzelnen genannt:

1-Cyclopropyl-6-fluor-1,4-dihydro-7-(4-methyl-3-phenyl-1-piperazinyl)-4-oxo-3-chinolincarbonsäure,
7-(4-Butyl-3-phenyl-1-piperazinyl)-1-cyclopropyl-6-fluor-1,4-dihydro-4-oxo-3-chinolincarbonsäure,
1-Cyclopropyl-6-fluor-1,4-dihydro-7-[4-(2-hydroxy-ethyl)-3-phenyl-1-piperazinyl]-4-oxo-3-chinolincarbonsäure,
1-Cyclopropyl-6-fluor-1,4-dihydro-7-[4-(2-methoxy-ethyl)-3-phenyl-1-piperazinyl]-4-oxo-3-chinolincarbonsäure,
7-[4-(2-Amino-ethyl)-3-phenyl-1-piperazinyl]-1-cyclopropyl-6-fluor-1,4-dihydro-4-oxo-3-chinolincarbonsäure, 1-Cyclopropyl-6-fluor-1,4-dihydro-7-[4-(2-dimethylaminoethyl)-3-phenyl-1-piperazinyl]-4-oxo-3-chinolincarbonsäure,
1-Cyclopropyl-6-fluor-7-[4-(2-fluor-ethyl)-3-phenyl-1-piperazinyl]-1,4-dihydro-4-oxo-3-chinolincarbonsäure,
7-[4-(2-Cyano-ethyl)-3-phenyl-1-piperazinyl]-1-cyclopropyl-6-fluor-1,4-dihydro-4-oxo-3-chinolincarbonsäure,
1-Cyclopropyl-6-fluor-1,4-dihydro-7-[4-(2-methoxycarbonylethyl)-3-phenyl-1-piperazinyl]-4-oxo-3-chinolincarbonsäure,
1-Cyclopropyl-6-fluor-1,4-dihydro-4-oxo-7-(4-phenacyl-3-phenyl-1-piperazinyl]-3-chinolincarbonsäure,
1-Cyclopropyl-6-fluor-7-(4-formyl-3-phenyl-1-piperazinyl)-1,4-dihydro-4-oxo-3-chinolincarbonsäure,
7-(4-Acetyl-3-phenyl-1-piperazinyl)-1-cyclopropyl-6-fluor-1,4-dihydro-4-oxo-3-chinolincarbonsäure,
1-Cyclopropyl-6-fluor-1,4-dihydro-7-[3-(4-methyl-cyclohexyl)-1-piperazinyl]-4-oxo-3-chinolincarbonsäure,
1-Cyclopropyl-6-fluor-1,4-dihydro-7-[3-(3,4,5-trimethoxyphenyl)-1-piperazinyl]-4-oxo-3-chinolincarbonsäure,
1-Cyclopropyl-6-fluor-1,4-dihydro-7-[3-(3,4-methylendioxy-phenyl)-1-piperazinyl]-4-oxo-3-chinolincarbonsäure,
7-[3-(4-Cyano-phenyl)-1-piperazinyl]-1-cyclopropyl-6-fluor-1,4-dihydro-4-oxo-3-chinolincarbonsäure,
7-[3-(3-Amino-phenyl)-1-piperazinyl]-1-cyclopropyl-6-fluor-1,4-dihydro-4-oxo-3-chinolincarbonsäure,
1-Cyclopropyl-6-fluor-1,4-dihydro-7-[3-(4-hydroxy-cyclohexyl)-1-piperazinyl]-4-oxo-3-chinolincarbonsäure,
7-[3-(3-Amino-cyclohexyl)-1-piperazinyl]-1-cyclopropyl-6-fluor-1,4-dihydro-4-oxo-3-chinolincarbonsäure,
1-Cyclopropyl-7-(4-ethyl-3-phenyl-1-piperazinyl)-6,8-difluor-1,4-dihydro-4-oxo-3-chinolincarbonsäure,
1-Cyclopropyl-6,8-difluor-7-[3-(4-fluorphenyl)-4-(3-oxobutyl)-1-piperazinyl-1,4-dihydro-4-oxo-3-chinolincarbonsäure,
1-Cyclopropyl-6,8-difluor-7-[4-formyl-3-(2-thienyl)-1-piperazinyl]-1,4-dihydro-4-oxo-3-chinolincarbonsäure.

Die erfindungsgemässen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemässen Verbindungen sind gegen ein sehr breites Spektrum von Mikroorganismen wirksam. Mit ihrer Hilfe können gramnegative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemässen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staph. aureus, Staph. Epidermidis (Staph. = Staphylococcus); Lactobacteriaceae, wie Streptokokken, z.B. Streptococcus pyogenes, α- bzw. β-hämolysierende Streptokokken, nicht γ-hämolysierende Streptokokken, Enterokokken und Diplococcus pneumoniae (Pneumokokken) Enterobacteriaceae, wie Escherichiae-Bakterien der Coli-Gruppe: Escherichia-Bakterien, z.B. Escherichia coli, Enterobacter-Bakterien, z.B. E. aerogenes, E. cloacae, (E. = Enterobacter), Klebsiella-Bakterien, z.B. K. pneumoniae, (K. = Klebsiella) Serratia, z.B. Serratia marcescens, Proteae-Bakterien der Proteus-Gruppe: Proteus, z.B. Pr. vulgaris, Pr. morganii, Pr. rettgeri, Pr. mirabilis (Pr. = Proteus); Pseudomonadaceae, wie Pseudomonas-Bakterien, z.B. Ps. aeruginosa (Ps. = Pseudomonas); Bacteroidaceae, wie Bacteroides-Bakterien, z.B. Bacteroides fragilis; Mykoplasmen, z.B. Mykoplasma pneumonia; ausserdem Mykobakterien, z.B. Mycobacterium tuberculosis, Mycobacterium leprae und atypische Mycobakterien.

Die obige Aufzählung von Erregern ist lediglich beispielhaft und keineswegs beschränkend aufzufassen.

Als Krankheiten, die durch die erfindungsgemässen Verbindungen verhindert, gebessert und/oder geheilt werden können, seien beispielsweise genannt:

Otitis; Pharyngitis; Pneumonie; Peritonitis; Pyelonephritis; Cystitis; Endocarditis; Systeminfektionen; Bronchitis; Arthritis; lokale Infektionen, septische Erkrankungen.

Zur Erfindung gehören pharmazeutische Zubereitungen, die neben nicht-toxischen, inerten pharmazeutisch geeigneten Trägerstoffen einen oder mehrere erfindungsgemässe Verbindungen enthalten oder die aus einem oder mehreren erfindungsgemässen Wirkstoffen bestehen, sowie Verfahren zur Herstellung dieser Zubereitungen.

Zur Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu versehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Rizinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl, und Süssmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.%, der Gesamtmischung vorhanden sein.

Die oben aufgeführten pharmazeutischen Zubereitungen können ausser den erfindungsgemässen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 0,5 bis etwa 500, vorzugsweise 1 bis 50 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemässen Wirkstoffe, vorzugsweise in Mengen von etwa 0,5 bis etwa 250, insbesondere 1 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obgenannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die erfindungsgemässen Verbindungen sind ebenfalls zur Vorbeugung und Behandlung von bakteriellen Infektionen bei Fischen geeignet.

Aus J. Med. Chem. 23, 1358 (1980) ist bereits bekannt, dass 1-Ethyl-6-fluor-1,4-dihydro-4-oxo-7-(1-piperazinyl)-3-chinolincarbonsäure (Norfloxacin) antibakterielle Eigenschaften besitzt. Die erfindungsgemässen Verbindungen sind jedoch dem Norfloxacin überlegen, wie aus der nachstehenden Tabelle, in der die MHK-Werte einiger der erfindungsgemässen Verbindungen und des Norfloxacins angegeben sind, hervorgeht.

Tabelle: MHK-Werte (Agardilutionstest/Isosensitestagar)

| Beispiel / Stamm | 1 | 2 | 3 | 5 | 8 | 11 | 12 | 14 | 16 | Norfloxacin |
|---|---|---|---|---|---|---|---|---|---|---|
| E. coli Neumann | 0.015 | 0.015 | 0.03 | 0.03 | ./. | ./. | 0.015 | 0.015 | 0.015 | 0.125 |
| Klebsiella 8085 | 0.015 | 0.015 | 0.03 | 0.03 | ./. | ./. | 0.015 | 0.03 | 0.015 | 0.25 |
| Klebsiella 6179 | 0.015 | 0.06 | 0.125 | 0.125 | ./. | ./. | 0.125 | 0.25 | 0.015 | 1 |
| Klebsiella 57 USA | 0.06 | 0.25 | 0.5 | 0.5 | ./. | ./. | 0.5 | 0.125 | 0.125 | 1 |
| Providencia 12052 | 4 | 8 | 8 | 8 | ./. | ./. | 8 | 32 | 4 | 0128 |
| Serratia 16040 | 8 | 16 | 16 | 16 | ./. | ./. | 16 | 8 | 2 | 32 |
| Staphylococcus FK 422 | 0.015 | 0.03 | 0.06 | 0.06 | 0.015 | 0.125 | 0.06 | 0.125 | 0.015 | 2 |
| Staphylococcus 1756 | 0.015 | 0.03 | 0.06 | 0.03 | 0.015 | 0.06 | 0.03 | 0.5 | 0.015 | 1 |
| Staphylococcus 133 | 0.015 | 0.06 | 0.06 | 0.06 | 0.03 | 0.06 | 0.03 | 0.125 | 0.015 | 1 |

Herstellungsbeispiele

Herstellung der Ausgangsprodukte (II)

Beispiel A

1-Cyclopropyl-6,7,8-trifluor-1,4-dihydro-4-oxo-3-chinolin-carbonsäure

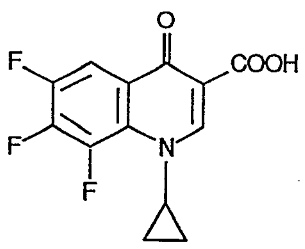

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml abs. Ethanol und 400 ml wasserfreiem Toluol bei 50–60°C zu. Dann wird noch 1 Stunde auf 50–60°C erhitzt, mit Trockeneis/Aceton auf -5°C bis -10°C gekühlt und bei dieser Temperatur eine Lösung von 212,5 g 2,3,4,5-Tetrafluorbenzoylchlorid in 80 ml abs. Toluol langsam zugetropft. Man rührt 1 Stunde bei 0 bis -5°C, lässt über Nacht auf Raumtemperatur kommen und lässt unter Eiskühlung ein Gemisch von 400 ml Eiswasser und 25 ml konz. Schwefelsäure zulaufen. Die Phasen werden getrennt und zweimal mit Toluol nachextrahiert. Die vereinigten Toluollösungen werden mit gesättigter NaCl-Lösung gewaschen, mit $Na_2SO_4$ getrocknet und das Lösungsmittel im Vakuum abgezogen. Man erhält 335 g 2,3,4,5-Tetra-fluorbenzoyl-malonsäurediethylester als Rohprodukt.

Eine Emulsion von 284,8 g rohem 2,3,4,5-Tetrafluorbenzoyl-malonsäurediethylester in 300 ml Wasser wird mit 0,3 g p-Toluolsulfonsäure versetzt. Man erhitzt unter gutem Rühren 5 Stunden zum Sieden, extrahiert die erkaltete Emulsion mehrmals mit Methylenchlorid, wäscht die vereinigten Methylenchlorid-Lösungen einmal mit gesättigter NaCl-Lösung, trocknet mit $Na_2SO_4$ und destilliert das Lösungsmittel im Vakuum ab. Die Fraktionierung des Rückstandes im Hochvakuum liefert 160,2 g 2,3,4,5-Tetrafluorbenzoyl-essigsäureethylester vom Siedepunkt 100–110°C/ 0,09–01 mbar. Schmelzpunkt 47–49°C.

Ein Gemisch von 110,7 g 2,3,4,5-Tetrafluorbenzoylessigsäureethylester 93,5 Orthoameisensäureethylester und 107 g Essigsäureanhydrid wird 2 Stunden auf 150°C erhitzt. Dann werden im Wasserstrahlvakuum und zuletzt im Hochvakuum bei einer Badtemperatur von ~ 120°C die flüchtigen Bestandteile abdestilliert. Zurück bleiben 123,9 g roher 2-(2, 3, 4, 5-Tetrafluorbenzoyl)-3-ethoxy-acrylsäureethylester. Er ist genügend rein für die weiteren Umsetzungen.

Eine Lösung von 123,9 g 2-(2,3,4,5-Tetrafluorbenzoyl)-3-ethoxyacrylsäureethylester in 250 ml Ethanol wird unter Eiskühlung und Rühren tropfenweise mit 23,2 g Cyclopropylamin versetzt. Wenn die exotherme Reaktion abgeklungen ist, wird noch 1 Stunde bei Raumtemperatur gerührt, das Lösungsmittel im Vakuum abgezogen und der Rückstand aus Cyclohexan/Petrolether umkristallisiert. Man erhält 115 g 2-(2,3,4,5-Tetrafluorbenzoyl)-3-cyclopropylamino-acrylsäureethylester vom Schmelzpunkt 63–65°C.

Eine Lösung von 107,8 g 2-(2,3,4,5-Tetrafluorbenzoyl)-3-cyclopropylamino-acrylsäureethylester in 400 ml wasserfreiem Dimethylformamid wird mit 21,2 g Natriumfluorid versetzt. Dann wird 2 Stunden unter Rückfluss gerührt und das Reaktionsgemisch heiss auf Eis gegossen. Der Niederschlag wird abgesaugt, mit Wasser gut gewaschen und im Vakuum über Calciumchlorid bei 100°C getrocknet. Man erhält 91,2 g 1-Cyclopropyl-6,7,8-trifluor-1,4-dihydro-4-oxo-3-chinolincarbonsäureethylester vom Schmelzpunkt 167–168°C.

Ein Gemisch von 94 g 1-Cyclopropyl-6,7,8-trifluor-1,4-dihydro-4-oxo-3-chinolincarbonsäureethylester, 600 ml Eisessig, 450 ml Wasser und 70 ml konz. Schwefelsäure wird 1,5 Stunden auf Rückfluss erhitzt. Dann giesst man die heisse Suspension auf Eis, saugt den Niederschlag ab, wäscht gut mit Wasser nach und trocknet im Vakuum bei 100°C. Man erhält auf diese Weise 88,9 g reine 1-Cyclopropyl-6,7,8-trifluor-1,4-dihydro-4-oxo-3-chinolincarbonsäure II ($X^1 = X^2 = F$) vom Schmelzpunkt 228–230°C (Z).

Beispiel B

1-Cyclopropyl-6,7-difluor-1,4-dihydro-4-oxo-3-chinolin-carbonsäure

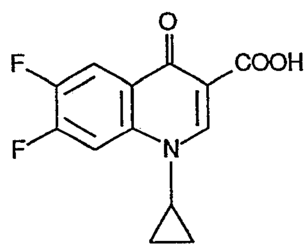

Ausgehend von 2,4,5-Trifluorbenzoylfluorid verfährt man analog Beispiel A, wobei folgende Stufen durchlaufen werden: 2,4,5-Trifluorbenzoylessigsäurediethylester (Siedepunkt: 92–95°C/ 0,5 mbar; Schmelzpunkt: 53–55°) → 2-(2,4,5-Trifluorbenzoyl)-3-ethoxy-acrylsäureethylester (Öl) → 2-(2,4,5-Trifluorbenzoyl)-3-cyclopropylamino-acrylsäureethylester (Öl) → 1-Cyclopropyl-6,7-difluor-1,4-dihydro-4-oxo-3-chinolincarbonsäureethylester (Schmelzpunkt: 230–233°) → 1-Cyclopropyl-6,7-difluor-1,4-dihydro-4-oxo-3-chinolincarbonsäure (Schmelzpunkt: 302–303° unter Zersetzung).

Herstellung der Wirkstoffe (I)

Beispiel 1

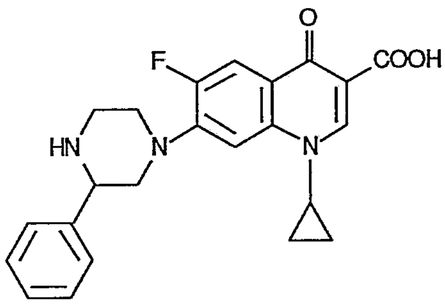

Eine Mischung aus 2,8 g (0,01 mol) 7-Chlor-1-cyclopropyl-6-fluor-1,4-dihydro-4-oxo-3-chinolincarbonsäure, 1,8 g (0,011 mol) 2-Phenyl-piperazin und 2,2 g (0,02 mol) 1,4-Diazabicyclo[2,2,2]octan in 6 ml Dimethylsulfoxid wird 4 Stunden auf 140°C erhitzt. Die Lösung wird im Hochvakuum eingeengt, der Rückstand mit 20 ml Wasser verrührt und mit 2n Salzsäure auf pH 7 eingestellt. Der Niederschlag wird abgesaugt, mit Wasser und Methanol gewaschen und in 30 ml Methanol aufgekocht. Man erhält 1,3 g (32% der Theorie) 1-Cyclopropyl-6-fluor-1,4-dihydro-4-oxo-7-(3-phenyl-1-piperazinyl)-3-chinolincarbonsäure vom Schmelzpunkt 218–220° (unter Zersetzung) (umkristallisiert aus Glykolmonomethylether).

Dasselbe Produkt wird erhalten, wenn man entsprechend 1-Cyclopropyl-6,7-difluor-1,4-dihydro-4-oxo-3-chinolincarbonsäure mit 2-Phenylpiperazin umsetzt.

Analog Beispiel 1 werden unter Verwendung der entsprechenden Piperazine die folgenden Verbindungen erhalten:

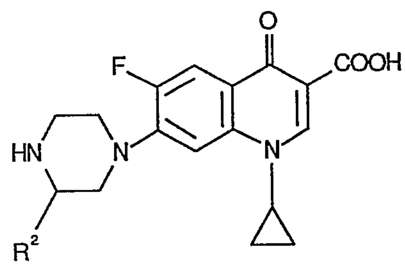

| Beispiel | $R^2$ | Schmelzpunkt (unter Zersetzung), °C |
|---|---|---|
| 2 | 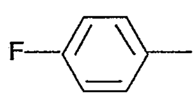 | 198–203 |
| 3 | 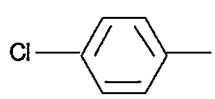 | 207–209 (aus Methanol) |
| 4 | 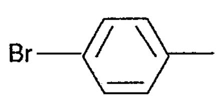 | 252–255 |
| 5 | 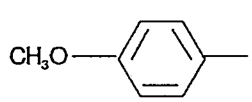 | 208–211 (aus Ethanol) |

| Beispiel | $R^2$ | Schmelzpunkt (unter Zersetzung), °C |
|---|---|---|
| 6 | 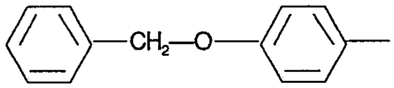 | 208–211 (aus Methanol) |
| 7 | 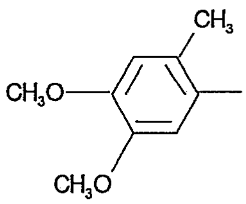 | 233–237 (aus Glykolmonomethylether) |
| 8 | 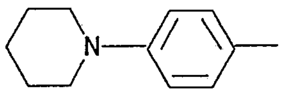 | 156–161 |
| 9 | 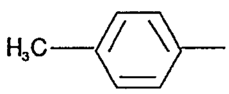 | 258–261 |
| 10 | 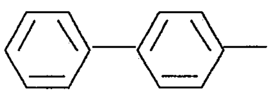 | 278–281 |
| 11 | 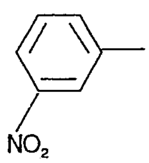 | 247–250 (aus Methanol) |
| 12 | 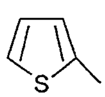 | 218–222 (aus Acetonitril) |
| 13 | 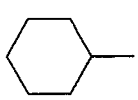 . HCl | 316–320 (aus Methanol) |

Beispiel 14

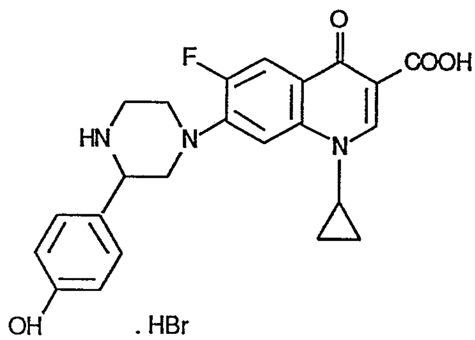

2,5 g (4,9 mmol) des Produktes aus Beispiel 6 in 30 ml Ethanol werden mit 60 ml 48%iger Bromwasserstoffsäure versetzt und 1 Stunde bei 55° gerührt. Die Mischung wird im Vakuum eingeengt, der Rückstand mit 50 ml Wasser verrührt, der Niederschlag abgesaugt und mit Methanol gewaschen. Man erhält 1,5 g (66% der Theorie) 1-Cyclopropyl-6-fluor-1,4-dihydro-7-[3-(4-hydroxy-phenyl)-1-piperazinyl]-4-oxo-3-chinolincarbonsäure-Hydrobromid vom Schmelzpunkt 295–298° (unter Zersetzung).

Beispiel 15

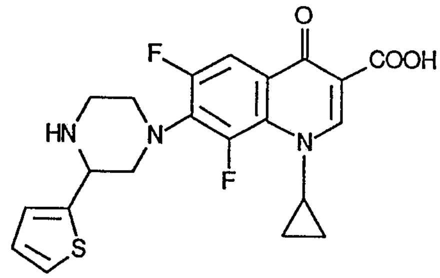

2,8 g (0,01 mol) 1-Cyclopropyl-6,7,8-trifluor-1,4-dihydro-4-oxo-3-chinolincarbonsäure (Beispiel A) werden mit 1,8 g (0,011 mol) 2-(2-Thienyl)-piperazin und 2,2 g (0,02 mol) 1,4-Diazabicyclo-[2,2,2]octan in 6 ml DMSO 2 Stunden auf 140°C erhitzt. Die Mischung wird im Hochvakuum eingeengt, der Rückstand mit 20 ml Wasser verrührt, der Niederschlag abgesaugt und in 20 ml Methanol aufgekocht. Es werden 2,4 g (56% der Theorie) 1-Cyclopropyl-6,8-difluor-1,4-dihydro-4-oxo-7-[3-(2-thienyl)-1-piperazinyl]-3-chinolincarbonsäure vom Schmelzpunkt 252–254°C (unter Zersetzung) erhalten. Nach der Umkristallisation aus Glykolmonomethylether bleibt der Schmelzpunkt unverändert.

Analog Beispiel 15 werden die folgenden Verbindungen erhalten:

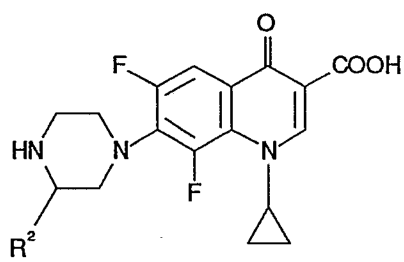

| Beispiel | $R^2$ | Schmelzpunkt (unter Zersetzung), °C |
|---|---|---|
| 16 | 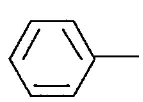 | 245–247 |
| 17 | 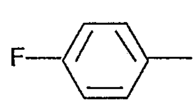 | 244–245 |
| 18 | 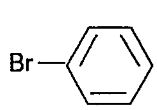 | 242–245 |

## Beispiel 19

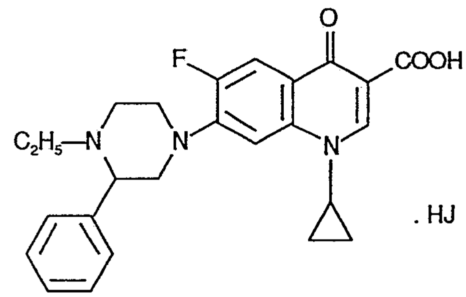

1,1 g (2,7 mmol) des Produktes aus Beispiel 1 werden in 10 ml Dimethylformamid mit 0,6 g Triethylamin und 0,9 g Ethyljodid 3 Stunden auf 80° erhitzt. Das Reaktionsgemisch wird im Vakuum eingeengt, der Rückstand mit 10 ml Wasser verrührt und aus Glykolmonomethylether umkristallisiert. Man erhält 0,75 g (49% der Theorie) 1-Cyclopropyl-7-(4-ethyl-3-phenyl-1-piperazinyl)-6-fluor-1,4-dihydro-4-oxo-3-chinolincarbonsäure-Hydrojodid vom Schmelzpunkt 240–243° (unter Zersetzung).

## Beispiel 20

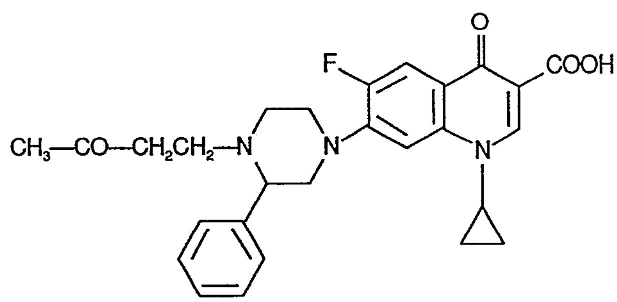

1,1 g (2,7 mmol) des Produktes aus Beispiel 1 werden in 15 ml Ethanol mit 1,05 g Methyl-vinylketon 8 Stunden unter Rückfluss erhitzt. Der Niederschlag wird abgesaugt und mit Ethanol gewaschen. Man erhält 0,9 g (70% der Theorie) 1-Cyclopropyl-6-fluor-1,4-dihydro-4-oxo-7-[4-(3-oxobutyl)-3-phenyl-1-piperazinyl]-3-chinolincarbonsäure vom Schmelzpunkt 224–226° (unter Zersetzung).

## Beispiel 21

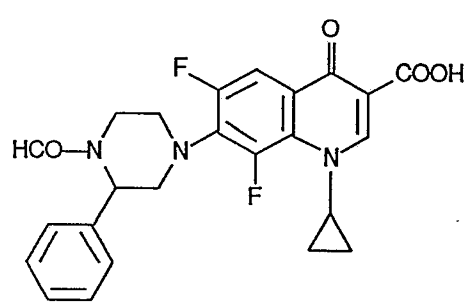

0,8 g (1,9 mmol) der Verbindung aus Beispiel 16 werden in 6 ml Dimethylformamid mit 0,6 ml 98–100%iger Ameisensäure 8 Stunden unter Rückfluss erhitzt. Das Reaktionsgemisch wird im Vakuum eingeengt, mit 20 ml Wasser verrührt und mit 5%iger Natriumhydrogencarbonat-Lösung auf pH5 eingestellt. Der ausgefallene Niederschlag wird abgesaugt, mit Wasser und Methanol gewaschen und aus Glykolmonomethylether umkristallisiert. Man isoliert 0,6 g (70% der Theorie) 1-Cyclopropyl-6,8-difluor-7-(4-formyl-3-phenyl-1-piperazinyl)-1,4-dihydro-4-oxo-3-chinolincarbonsäure vom Schmelzpunkt 242–245° (unter Zersetzung).

Beispiel für eine erfindungsgemässe Tablette

| | |
|---|---|
| Jede Tablette enthält: | |
| Verbindung des Beispiels | 291,5 mg |
| Mikrokristalline Cellulose | 27,5 mg |
| Maisstärke | 36,0 mg |
| Poly(1-vinyl-2-pyrrolidon) unlöslich | 15,0 mg |
| Hochdisperses Siliciumdioxid | 2,5 mg |
| Magnesiumstearat | 2,5 mg |
| | 375,0 mg |
| Die Lackhülle enthält: | |
| Poly (O-hydroxypropyl-O-methyl)cellulose 15 cp (Hydroxypropyl Methylcellulose USP) | 3,9 mg |
| Macrogol 4000 rec. INN (Polyethylenglykole DAB) | 1,3 mg |
| Titan (IV)-oxid (Titanium Dioxide BP) | 1,3 mg |
| | 6,5 mg |

**Patentansprüche**

1. 1-Cyclopropyl-6-fluor-1,4-dihydro-4-oxo-7-(1-piper-azinyl)-3-chinolincarbonsäuren der Formel (I)

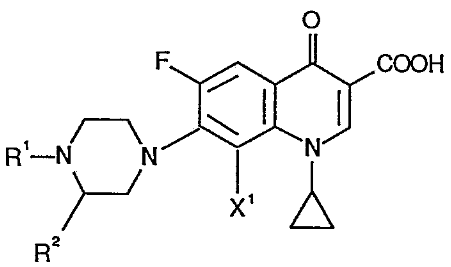

(I)

in welcher
$R^1$ Wasserstoff, gegebenenfalls durch Hydroxy, Methoxy, Amino, Dimethylamino, Halogen, Cyano oder Alkoxycarbonyl mit ein oder zwei Kohlenstoffatomen im Alkylteil substituiertes geradkettiges oder verzweigtes Alkyl mit 1 bis 4 Kohlenstoffatomen, Oxoalkyl mit bis zu 4 Kohlenstoffatomen, Phenacyl, Acyl mit 1 bis 4 Kohlenstoffatomen,
$R^2$ gegebenenfalls durch Halogen, Methyl, Phenyl, Cyano, Hydroxy, Methoxy, Benzyloxy, Amino, Methylamino, Dimethylamino, Piperidino oder Nitro ein bis dreifach substituiertes Phenyl sowie Cyclohexyl, Methylendioxyphenyl, Methylendioxycyclohexyl, Thienyl, und
$X^1$ Wasserstoff oder Fluor bedeuten, und deren pharmazeutisch verwendbare Hydrate, Säureadditionssalze, Alkali- und Erdalkalisalze.

2. 1-Cyclopropyl-6-fluor-1,4-dihydro-4-oxo-7-(1-piperazinyl)-3-chinolincarbonsäuren der Formel (I) in Anspruch 1, in welcher
$R^1$ Wasserstoff, gegebenenfalls durch Hydroxy, Methoxy, Halogen, Cyano oder Alkoxycarbonyl mit ein oder zwei Kohlenstoffatomen im Alkylteil substituiertes geradkettiges oder verzweigtes Alkyl mit 1 bis 4 Kohlenstoffatomen, Oxoalkyl mit bis zu 4 Kohlenstoffatomen, Phenacyl, Formyl oder Acetyl,
$R^2$ gegebenenfalls durch Chlor, Brom, Fluor, Methyl, Phenyl, Cyano, Hydroxy, Methoxy, Benzyloxy, Amino, Piperidino oder Nitro ein bis dreifach substituiertes Phenyl sowie Cyclohexyl oder Thienyl und
$X^1$ Wasserstoff oder Fluor bedeuten.

3. 1-Cyclopropyl-6-fluor-1,4-dihydro-4-oxo-7-(1-piperazinyl)-3-chinolincarbonsäuren der Formel (I) in Anspruch 1, in welcher
$R^1$ Wasserstoff, gegebenenfalls durch Hydroxy, Methoxy, Cyano oder Alkoxycarbonyl mit ein oder zwei Kohlenstoffatomen im Alkylteil substituiertes geradkettiges oder verzweigtes Alkyl mit 1 bis 3 Kohlenstoffatomen, Oxoalkyl mit bis zu 4 Kohlenstoffatomen, Phenacyl, Formyl oder Acetyl,
$R^2$ gegebenenfalls durch Chlor, Brom, Fluor, Methyl, Phenyl, Hydroxy, Methoxy, Benzyloxy, Amino, Piperidino oder Nitro ein- bis dreifach substituiertes Phenyl sowie Cyclohexyl oder Thienyl und
$X^1$ Wasserstoff oder Fluor bedeuten.

4. Verbindungen der Formel (I) in Anspruch 1 zur Anwendung in einem Verfahren zur Bekämpfung von Erkrankungen des menschlichen oder tierischen Körpers.

5. Verfahren zur Herstellung von Verbindungen der Formel (I)

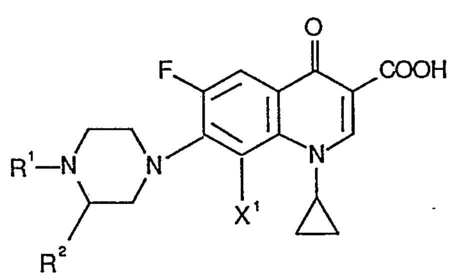

(I)

in welcher
$R^1$ Wasserstoff, gegebenenfalls durch Hydroxy,

Methoxy, Amino, Dimethylamino, Halogen, Cyano oder Alkoxy-carbonyl mit ein oder zwei Kohlenstoffatomen im Alkylteil substituiertes geradkettiges oder verzweigtes Alkyl mit 1 bis 4 Kohlenstoffatomen, Oxoalkyl mit bis zu 4 Kohlenstoffatomen, Phenacyl, Acyl mit 1 bis 4 Kohlenstoffatomen,
$R^2$ gegebenenfalls durch Halogen, Methyl, Phenyl, Cyano, Hydroxy, Methoxy, Benzyloxy, Amino, Methylamino, Dimethylamino, Piperidino oder Nitro ein bis dreifach substituiertes Phenyl sowie Cyclohexyl, Methylendioxyphenyl, Methylendioxycyclohexyl, Thienyl, und
$X^1$ Wasserstoff oder Fluor bedeuten, und deren pharmazeutisch verwendbare Hydrate, Säureadditionssalze, Alkali- und Erdalkalisalze, dadurch gekennzeichnet, dass man entweder
a) eine 1-Cyclopropyl-6-fluor-1,4-hydro-4-oxo-3-chinolincarbonsäure der Formel (II)

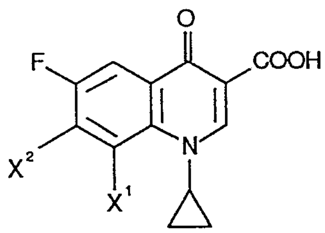

(II)

in welcher $X^1$ die oben angegebene Bedeutung hat und $X^2$ für Halogen, insbesondere Fluor oder Chlor steht, mit Piperazin der Formel (III)

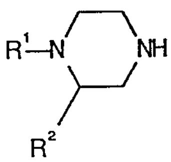

(III)

in welcher $R^1$ und $R^2$ die oben angegebene Bedeutung haben, gegebenenfalls in Gegenwart von Säurebindern umsetzt, oder
b) eine 7-(1-Piperazinyl)-chinoloncarbonsäure der Formel (IV)

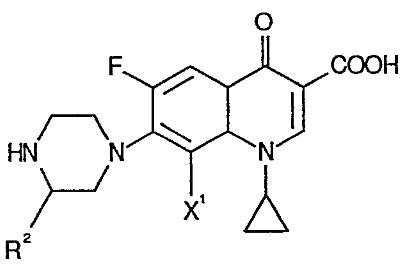

(IV)

in welcher $R^2$ und $X^1$ die oben angegebene Bedeutung haben, mit einer Verbindung der Formel (V)
$R^1$-X (V),
in welcher $R^1$ die oben angegebene Bedeutung hat, jedoch nicht Wasserstoff sein kann, und
X Fluor, Chlor, Brom, Jod, Hydroxy, Acyloxy, Phenoxy oder 4-Nitrophenoxy bedeutet, gegebenenfalls in Gegenwart von Säurebindern umsetzt, oder
c) eine 7-(1-Piperazinyl)-chinoloncarbonsäure der Formel (IV) mit einem Michael-Acceptor der Formel (VI)

$R^3$—CH=$CH_2$ (VI)

in der $R^3$ für CN, $CH_3CO$ oder $COOR^4$ steht, wobei $R^4$ Methyl oder Ethyl bedeutet, umsetzt.

6. Arzneimittel, enthaltend 1-Cyclopropyl-6-fluor-1,4-dihydro-4-oxo-7-(1-piperazinyl)-3-chinolincarbonsäuren der Formel (I)

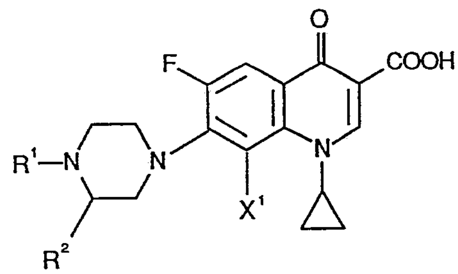

(I)

in welcher
$R^1$ Wasserstoff, gegebenenfalls durch Hydroxy, Methoxy, Amino, Dimethylamino, Halogen, Cyano oder Alkoxycarbonyl mit ein oder zwei Kohlenstoffatomen im Alkylteil substituiertes geradkettiges oder verzweigtes Alkyl mit 1 bis 4 Kohlenstoffatomen, Oxoalkyl mit bis zu 4 Kohlenstoffatomen, Phenacyl, Acyl mit 1 bis 4 Kohlenstoffatomen,
$R^2$ gegebenenfalls durch Halogen, Methyl, Phenyl, Cyano, Hydroxy, Methoxy, Benzyloxy, Amino, Methylamino, Dimethylamino, Piperidino oder Nitro ein bis dreifach substituiertes Phenyl sowie Cyclohexyl, Methylendioxyphenyl, Methylendioxycyclohexyl oder Thienyl, und
$X^1$ Wasserstoff oder Fluor bedeuten, und deren pharmazeutisch verwendbare Hydrate, Säureadditionssalze, Alkali- und Erdalkalisalze.

7. Verfahren zur Herstellung von Arzneimitteln, dadurch gekennzeichnet, dass man 1-Cyclopropyl-6-fluor-1,4-dihydro-4-oxo-7-(1-piperazinyl)-3-chinolincarbonsäuren der Formel (I)

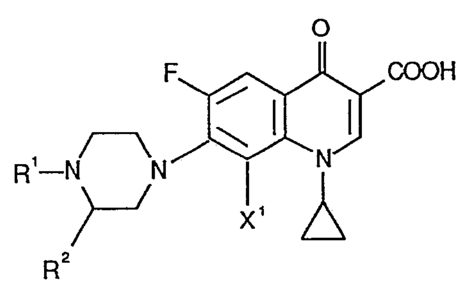

(I)

in welcher
$R^1$ Wasserstoff, gegebenenfalls durch Hydroxy, Methoxy, Amino, Dimethylamino, Halogen, Cyano oder Alkoxycarbonyl mit ein oder zwei Kohlenstoffatomen im Alkylteil substituiertes geradkettiges oder verzweigtes Alkyl mit 1 bis 4 Kohlenstoffatomen, Oxoalkyl mit bis zu 4 Kohlenstoffatomen, Phenacyl, Acyl mit 1 bis 4 Kohlenstoffatomen.

$R^2$ gegebenenfalls durch Halogen, Methyl, Phenyl, Cyano, Hydroxy, Methoxy, Benzyloxy, Amino, Methylamino, Dimethylamino, Piperidino oder Nitro, ein bis dreifach substituiertes Phenyl sowie Cyclohexyl, Methylendioxyphenyl, Methylendioxycyclohexyl oder Thienyl, und

$X^1$ Wasserstoff oder Fluor bedeuten, oder deren pharmazeutisch verwendbare Hydrate, Säureadditionssalze, Alkali- und Erdalkalisalze, mit inerten, nichtionischen, pharmazeutisch geeigneten Trägerstoffen vermischt.

8. Verwendung von 1-Cyclopropyl-6-fluor-1,4-dihydro-4-oxo-7-(1-piperazinyl)-3-chinolincarbonsäuren der Formel (I) gemäss Anspruch 1 zur Herstellung von Arzneimitteln.

## Claims

1. 1-Cyclopropyl-6-fluoro-1,4-dihydro-4-oxo-7-(1-piperazinyl)-3-quinolinecarboxylic acids of the formule (I)

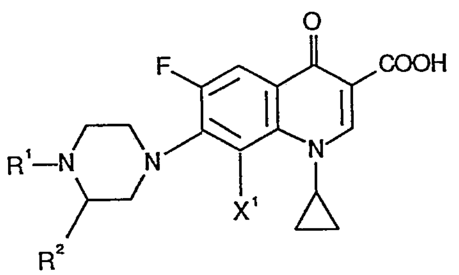

(I)

in which

$R^1$ denotes hydrogen, straight-chain or branched alkyl which has 1 to 4 carbon atoms and is optionally substituted by hydroxyl, methoxy, amino, dimethylamino, halogen, cyano or alkoxycarbonyl having one or two carbon atoms, in the alkyl moiety, oxoalkyl having up to 4 carbon atoms, phenacyl, or acyl having 1 to 4 carbon atoms,

$R^2$ denotes phenyl which is optionally singly to triply substituted by halogen, methyl, phenyl, cyano, hydroxyl, methoxy, benzyloxy, amino, methylamino, dimethylamino, piperidino or nitro, or cyclohexyl, methylenedioxyphenyl, methylenedioxycyclohexyl or thienyl, and

$X^1$ denotes hydrogen or fluorine, and their pharmaceutically utilisable hydrates, acid addition salts, alkali metal and alkaline earth metal salts.

2. 1-Cyclopropyl-6-fluoro-1,4-dihydro-4-oxo-7-(1-piperazinyl)-3-quinolinecarboxylic acids of the formula (I) in Claim 1, in which

$R^1$ denotes hydrogen, straight-chain or branched alkyl which has 1 to 4 carbon atoms and is optionally substituted by hydroxyl, methoxy, halogen, cyano or alkoxycarbonyl having one or two carbon atoms in the alkyl moiety, oxoalkyl having up to 4 carbon atoms, phenacyl, formyl or acetyl,

$R^2$ denotes phenyl which is optionally singly to triply substituted by chlorine, bromine, fluorine, methyl, phenyl, cyano, hydroxyl, methoxy, benzyloxy, amino, piperidino or nitro, or cyclohexyl or thienyl, and

$X^1$ denotes hydrogen or fluorine.

3. 1-Cyclopropyl-6-fluoro-1,4-dihydro-4-oxo-7-(1-piperazinyl)-3-quinolinecarboxylic acids of the formula (I) in Claim 1, in which

$R^1$ denotes hydrogen, straight-chain or branched alkyl which has 1 to 3 carbon atoms and is optionally substituted by hydroxyl, methoxy, cyano or alkoxycarbonyl having one or two carbon atoms in the alkyl moiety, oxoalkyl having up to 4 carbon atoms, phenacyl, formyl or acetyl,

$R^2$ denotes phenyl which is optionally singly to triply substituted by chlorine, bromine, fluorine, methyl, phenyl, hydroxyl, methoxy, benzyloxy, amino, piperidino or nitro, or cyclohexyl or thienyl, and

$X^1$ denotes hydrogen or fluorine.

4. Compounds of the formula (I) in Claim 1, for use in a process for combating illnesses of the human or animal body.

5. Process for the preparation of compounds of the formula (I)

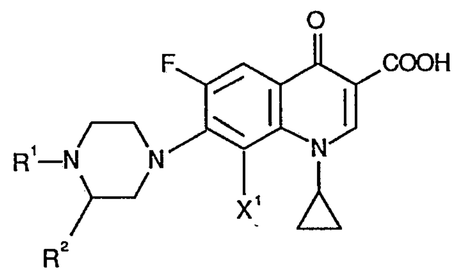

(I)

in which

$R^1$ denotes hydrogen, straight-chain or branched alkyl which has 1 to 4 carbon atoms and is optionally substituted by hydroxyl, methoxy, amino, dimethylamino, halogen, cyano or alkoxycarbonyl having one or two carbon atoms in the alkyl moiety, oxoalkyl having up to 4 carbon atoms, phenacyl, or acyl having 1 to 4 carbon atoms,

$R^2$ denotes phenyl which is optionally singly to triply substituted by halogen, methyl, phenyl, cyano, hydroxyl, methoxy, benzyloxy, amino, methylamino, dimethylamino, piperidino or nitro, or cyclohexyl, methylenedioxyphenyl, methylenedioxycyclohexyl or thienyl, and

$X^1$ denotes hydrogen or fluorine, and their pharmaceutically utilisable hydrates, acid addition salts, alkali metal and alkaline earth metal salts, characterised in that either

a) a 1-cyclopropyl-6-fluoro-1,4-dihydro-4-oxo-3-quinolinecarboxylic acid of the formula (II)

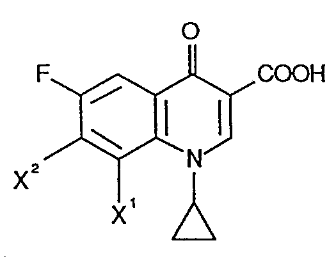

(II)

in which $X^1$ has the abovementioned meaning and $X^2$ represents halogen, in particular fluorine or chlorine, is reacted with a piperazine of the formula (III)

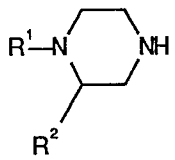

(III)

in which $R^1$ and $R^2$ have the abovementioned meaning, where appropriate in the presence of acid-binding agents, or

b) a 7-(1-piperazinyl)quinolonecarboxylic acid of the formula (IV)

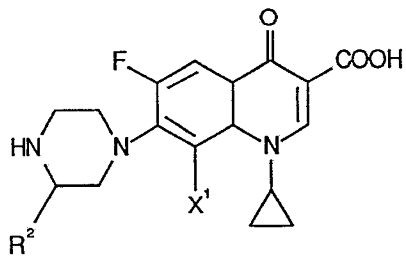

(IV)

in which $R^2$ and $X^1$ have the abovementioned meaning, is reacted with a compound of the formula (V)

$R^1$-X (V)

in which $R^1$ has the abovementioned meaning but cannot be hydrogen, and

X denotes fluorine, chlorine, bromine, iodine, hydroxyl, acyloxy, phenoxy or 4-nitrophenoxy, where appropriate in the presence of acid-binding agents, or

c) a 7-(1-piperazinyl)quinolonecarboxylic acid of the formula (IV) is reacted with a Michael acceptor of the formula (VI)

$R^3$-CH=$CH_2$ (VI)

in which $R^3$ represents CN, $CH_3CO$ or $COOR^4$, $R^4$ denoting methyl or ethyl.

6. Medicament containing 1-cyclopropyl-6-fluoro-1,4-dihydro-4-oxo-7-(1-piperazinyl)-3-quinolinecarboxylic acids of the formula (I)

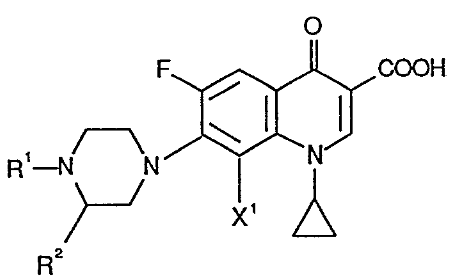

(I)

in wich

$R^1$ denotes hydrogen, straight-chain or branched alkyl which has 1 to 4 carbon atoms and is optionally substituted by hydroxyl, methoxy, amino, dimethylamino, halogen, cyano or alkoxycarbonyl having one or two carbon atoms in the alkyl moiety, oxoalkyl having up to 4 carbon atoms, phenacyl, or acyl having 1 to 4 carbon atom

$R^2$ denotes phenyl which is optionally singly to triply substituted by halogen, methyl, phenyl, cyano, hydroxyl, methoxy, benzyloxy, amino, methylamino, dimethylamino, piperidino or nitro, or cyclohexyl, methylenedioxyphenyl, methylenedioxycyclohexyl or thienyl, and

$X^1$ denotes hydrogen or fluorine, and their pharmaceutically utilisable hydrates, acid addition salts, alkali metal and alkaline earth metal salts.

7. Process for the preparation of medicaments, characterised in that 1-cyclopropyl-6-fluoro-1,4-dihydro-4-oxo-7-(1-piperazinyl)-3-quinolinecarboxylic acids of the formula (I)

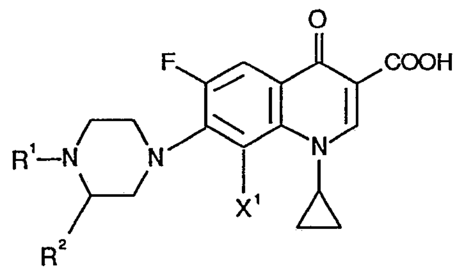

(I)

in which

$R^1$ denotes hydrogen, straight-chain or branched alkyl which has 1 to 4 carbon atoms and is optionally substituted by hydroxyl, methoxy, amino, dimethylamino, halogen, cyano or alkoxycarbonyl having one or two carbon atoms in the alkyl moiety oxoalkyl having up to 4 carbon atoms, phenacyl, or acyl having 1 to 4 carbon atoms,

$R^2$ denotes phenyl which is optionally singly to triply substituted by halogen, methyl, phenyl, cyano, hydroxyl, methoxy, benzyloxy, amino, methylamino, dimethylamino, piperidino or nitro, or cyclohexyl, methylenedioxyphenyl, methylenedioxycyclohexyl or thienyl, and

$X^1$ denotes hydrogen or fluorine, or their pharmaceutically utilisable hydrates, acid addition salts, alkali metal and alkaline earth metal salts, are mixed with inert, non-ionic pharmaceutically suitable vehicels.

8. Use of 1-cyclopropyl-6-fluoro-1,4-dihydro-4-oxo-7-(1-piperazinyl)-3-quinolinecarboxylic acids of the formula (I) according to Claim 1 for the preparation of medicaments.

## Revendications

1. Acides 1-cyclopropyl-6-fluoro-1,4-dihydro-4-oxo-7-(1-pipérazinyl)-3-quinoléine carboxyliques de la formule (I)

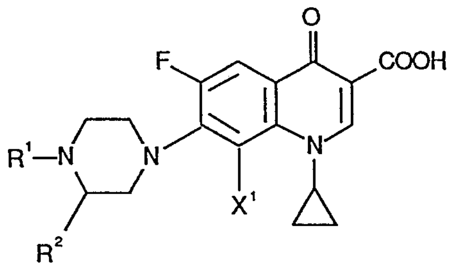

(I)

dans laquelle
$R^1$ représente l'hydrogène, un groupe alcyle à 1 à 4 atomes de carbone, phénacyle, oxoalkyle à 4 atomes de carbone au maximum, alkyle à 1 à 4 atomes de carbone à chaîne droite ou ramifiée, portant éventuellement un substituant hydroxy, méthoxy, amino, diméthylamino, halogéno, cyano ou alcoxycarbonyle à un ou deux atomes de carbone dans le radical alkyle,
$R^2$ représente un groupe thiényle, méthylènedioxycyclohexyle, méthylènedioxyphényle, cyclohexyle de même que phényle, portant éventuellement un à trois substituants halogéno, méthyle, phényle, cyano, hydroxy, méthoxy, benzyloxy, amino, méthylamino, diméthylamino, pipéridino ou nitro, et
$X^1$ représente l'hydrogène ou le fluor et leurs hydrates, sels d'addition d'acides, sels alcalins et sels alcalinoterreux utilisables en pharmacie.

2. Acides 1-cyclopropyl-6-fluoro-1, 4-dihydro-4-oxo-7-(1-pipérazinyl)-3-quinoléine carboxyliques de la formule (I) selon la revendication 1, dans laquelle
$R^1$ représente l'hydrogène, un groupe acétyle, formyle, phénacyle, oxoalkyle à 4 atomes de carbone au maximum ou alkyle à 1 à 4 atomes de carbone à chaîne droite ou ramifiée, portant éventuellement un substituant hydroxy, méthoxy, halogéno, cyano ou alcoxycarbonyle à un ou deux atomes de carbone dans le radical alkyle,
$R^2$ représente un groupe thiényle ou cyclohexyle de même que phényle, portant éventuellement un à trois substituants chlore, brome, fluor, méthyle, phényle, cyano, hydroxy, méthoxy, benzyloxy, amino, pipéridino ou nitro et
$X^1$ représente l'hydrogène ou le fluor.

3. Acides 1-cyclopropyl-6-fluoro-1,4-dihydro-4-oxo-7-(1-pipérazinyl)-3-quinoléine carboxyliques de la formule (I) selon la revendication 1, dans lesquels
$R^1$ représente l'hydrogène, un groupe acétyle, formyle, phénacyle, oxoalkyle à 4 atomes de carbone au maximum ou alkyle à 1 à 3 atomes de carbone à chaîne droite ou ramifiée, portant éventuellement un substituant hydroxy, méthoxy, cyano ou alcoxycarbonyle à un ou deux atomes de carbone dans le radical alkyle,
$R^2$ représente un groupe thiényle ou cyclohexyle de même que phényle portant éventuellement un à trois substituants chlore, brome, fluor, méthyle, phényle, hydroxy, méthoxy, benzyloxy, amino, pipéridino ou nitro
$X^1$ représente l'hydrogène ou le fluor.

4. Composés de la formule (I) selon la revendication 1, à utiliser dans une méthode de lutte contre les maladies du corps humain ou animal.

5. Procédé de production des composés de la formule (I)

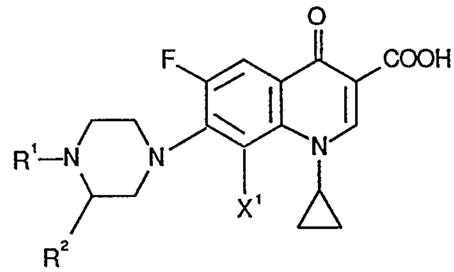

(I)

dans laquelle
$R^1$ représente l'hydrogène, un groupe acyle à 1 à 4 atomes de carbone, phénacyle, oxoalkyle à 4 atomes de carbone au maximum, alkyle à 1 à 4 atomes de carbone à chaîne droite ou ramifiée, portant éventuellement un substituant hydroxy, méthoxy, amino, diméthylamino, halogéno, cyano ou alcoxycarbonyle à un ou deux atomes de carbone dans le radical alkyle,
$R^2$ représente un groupe thiényle, méthylènedioxycyclohexyle, méthylènedioxyphényle, cyclohexyle de même que phényle portant éventuellement un à trois substituants halogéno, méthyle, phényle, cyano, hydroxy, méthoxy, benzyloxy, amino, méthylamino, diméthylamino, pipéridino ou nitro, et
$X^1$ représente l'hydrogène ou le fluor et leurs hydrates, sels d'addition d'acides, sels alcalins et alcalinoterreux utilisables en pharmacie, caractérisé en ce que l'on fait réagir soit
a) un acide 1-cyclopropyl-6-fluoro-1,4-dihydro-4-oxo-3-quinoléine carboxylique de la formule (II)

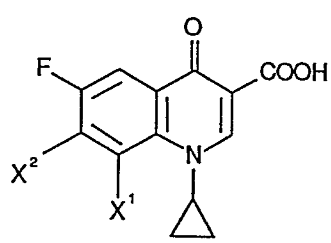

(II)

dans laquelle $X^1$ a la signification ci-dessus et $X^2$ représente un halogène et, en particulier, le fluor ou le chlore, avec une pipérazine de la formule (III)

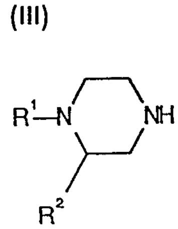

(III)

dans laquelle $R^1$ et $R^2$ ont les significations précitées, éventuellement en présence de fixateurs d'acides ou
b) un acide 7-(1-pipérazinyl)-quinoléine carboxylique de la formule (IV)

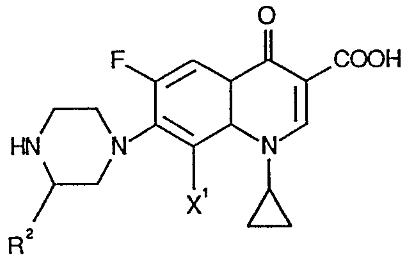

(IV)

dans laquelle $R^2$ et $X^1$ ont les significations mentionnées ci-dessus, avec un composé de la formule (V)

$R^1$-X (V)

dans laquelle $R^1$ a la signification précitée mais ne peut pas être de l'hydrogène et où
X représente le fluor, le chlore, le brome, l'iode, un groupe hydroxy, acyloxy, phénoxy ou 4-nitrophénoxy, éventuellement en présence de fixateurs d'acide ou
c) un acide 7--1-pipérazinyl)-quinoléine carboxylique de la formule (VI) avec un accepteur de Michael de la formule (VI)

$R^3-CH = CH_2$ (VI)

dans laquelle $R^3$ représente CN, $CH_3CO$ ou $COOR^4$, tandis que $R^4$ représente un radical méthyle ou éthyle.

6. Médicaments contenant les acides 1-cyclopropyl-6-fluoro-1,4-dihydro-4-oxo-7-(1-pipérazinyl)-3-quinoléine carboxyliques de la formule (I)

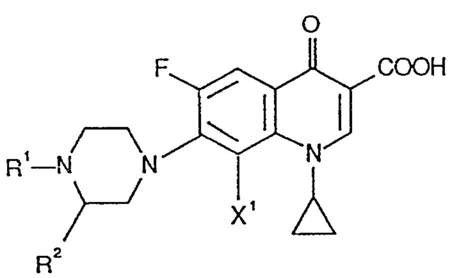

(I)

dans laquelle
$R^1$ représente l'hydrogène, un groupe acyle à 1 à 4 atomes de carbone, phénacyle, oxoalkyle à 4 atomes de carbone au maximum, alkyle à 1 à 4 atomes de carbone à chaîne droite ou ramifiée, portant éventuellement un substituant hydroxy, méthoxy, amino, diméthylamino, halogéno, cyano ou alcoxycarbonyle à un ou deux atomes de carbone, dans le radical alkyle
$R^2$ représente un groupe thiényle, méthylènedioxycyclohexyle, méthylènedioxyphényle, cyclohexyle ou phényle portant éventuellement un à trois substituants halogéno, méthyle, phényle, cyano, hydroxy, méthoxy, benzyloxy, amino, méthylamino, diméthylamino, pipéridino ou nitro,
$X^1$ représente l'hydrogène ou le fluor, et leurs hydrates, sels d'addition d'acides, sels alcalins et alcalinoterreux utilisables en pharmacie.

7. Procédé pour la production de médicaments, caractérisé en ce que l'on mélange les acides 1-cyclopropyl-6-fluoro-1,4-dihydro-4-oxo-7-(1-pipérazinyl)-3-quinoléine carboxyliques de la formule (I)

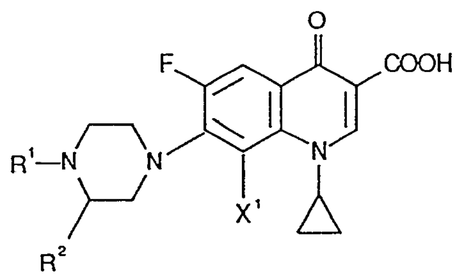

(I)

dans laquelle
$R^1$ représente l'hydrogène, un groupe acyle à 1 à 4 atomes de carbone, phénacyle, oxoalkyle à 4 atomes de carbone au maximum, alkyle à 1 à 4 atomes de carbone à chaîne droite ou ramifiée, portant éventuellement un substituant hydroxy, méthoxy, amino, diméthylamino, halogéno, cyano ou alcoxycarbonyle à un ou deux atomes de carbone dans le radical alkyle,
$R^2$ représente un groupe thiényle, méthylènedioxycyclohexyle, méthylènedioxyphényle, cyclohexyle de même que phényle portant éventuellement un à trois substituants halogèno, méthyle, phényle, cyano, hydroxy, méthoxy, benzyloxy, amino, méthylamino, diméthylamino, pipéridino ou nitro,
$X^1$ représente l'hydrogène ou le fluor ainsi que leurs hydrates, sels d'addition d'acides, sels alcalins et sels alcalinoterreux utilisables en pharmacie, avec des supports inertes non ioniques utilisables en pharmacie.

8. Utilisation des acides 1-cyclopropyl-6-fluoro-1,4-dihydro-4-oxo-7-(1-pipérazinyl)-3-quinoléine carboxylique de la formule (I) selon la revendication 1 pour la production de médicaments.